# EUROPEAN PATENT SPECIFICATION

(11) **EP 3 965 823 B1**
(45) Date of publication and mention of the grant of the patent: **11.03.2026**
(21) Application number: 19928273.2
(22) Date of filing: 07.05.2019
(51) Int. Cl.: A61K 33/242, A61K 47/00, A61P 3/06, A61K 9/00, A61K 9/51, A61K 47/69

(54) **USE OF GOLD NANOCLUSTERS IN TREATING HYPERCHOLESTEROLEMIA OR HYPERCHOLESTEROLEMIA-ASSOCIATED DISEASES**
VERWENDUNG VON GOLDNANOCLUSTERN ZUR BEHANDLUNG VON HYPERCHOLESTERINÄMIE ODER HYPERCHOLESTERINÄMIE-ASSOZIIERTEN ERKRANKUNGEN
UTILISATION DE NANOAGRÉGATS D'OR DANS LE TRAITEMENT DE L'HYPERCHOLESTÉROLÉMIE OU DE MALADIES ASSOCIÉES À L'HYPERCHOLESTÉROLÉMIE

(43) Date of publication of application: 16.03.2022
(73) Proprietor: Goldred Nanobiotech Co., Ltd., Taoyuan City 320 (TW); Yeh, Hung-i, Taipei City, Taiwan 104 (TW); Lee, Yi-Nan, New Taipei City, Taiwan 251 (TW); Wang, Hsueh-Hsiao, Taipei City, Taiwan 116 (TW)
(72) Inventor: CHANG, Hong-Shong, Chung-Li Tao-Yuan City, Taiwan 320 (CN); LI, Kuan-Jung, North Dist. Tainan City, Taiwan 704 (CN); LAI, Wei-Chung, Tamsui Dist. New Taipei City, Taiwan 251 (CN); YEH, Hung-i, Zhongshan Dist. Taipei City, Taiwan 104 (CN); LEE, Yi-Nan, Tamsui Dist. New Taipei City, Taiwan 251 (CN); WANG, Hsueh-Hsiao, Wenshan Dist. Taipei City, Taiwan 116 (CN)
(74) Representative: Lang, Christian
(86) International application number: PCT/CN2019/085810
(87) International publication number: WO 2020/223893

(56) References cited:
- EP-A1- 3 698 787
- US-B2- 9 101 672
- CHEN HUI ET AL: "Gold nanoparticles improve metabolic profile of mice fed a high-fat diet", vol. 16, no. 1, 1 December 2018 (2018-12-01), pages 11, XP055977334, Retrieved from the Internet <URL:https://jnanobiotechnology.biomedcentral.com/counter/pdf/10.1186/s12951-018-0338-1.pdf> DOI: 10.1186/s12951-018-0338-1
- IBRAHIM AMMAL ESMAEEL: "Effect of Gold Nanoparticles on Serum Lipid Profile in Albino Mice", ONLINE JOURNAL OF BIOLOGICAL SCIENCES, 1 April 2018 (2018-04-01), pages 432 - 434, XP055977253, Retrieved from the Internet <URL:https://thescipub.com/pdf/ojbsci.2018.432.434.pdf> [retrieved on 20221102]

## Description

### BACKGROUND OF THE INVENTION

### 1. FIELD OF THE INVENTION

The present disclosure in general relates to the field of disease treatment. More particularly, the present disclosure relates to the use of dihydrolipoic acid (DHLA)-coated gold nanoclusters in the treatment of hypercholesterolemia.

### 2. DESCRIPTION OF RELATED ART

Hypercholesterolemia, also known as high cholesterol, is a condition caused by high levels of cholesterol in the blood. Cholesterol is a substance vital for the formation of cell membranes, hormones, and compounds involving fat digestion. However, excess cholesterol would abnormally accumulate and form plaques or clots in blood vessels that leads to the development of atherosclerosis, a disease characterized in the thickening and loss of elasticity of the walls of blood vessels. Atherosclerosis is closely associated with the occurrence and progression of several cardiovascular diseases (CVDs), including coronary heart disease, angina, heart attack, stroke, transient ischaemic attack (TIA), peripheral arterial disease (PAD, *e.g.,* limb ischemia) and restenosis.

Statins and surgery are the mainstay of treatment for atherosclerosis. Statins are a class of lipid-lowering medications, which block the pathway for synthesizing cholesterol in the liver via inhibiting the function of 3-hydroxy-3-methyl-glutaryl-coenzyme A reductase (HMG-CoA reductase). As a prescription for the treatment of atherosclerosis, statins are usually accompanied with adverse side-effects, such as dizziness, nose bleeding, sore throat, headache, constipation, diarrhea, indigestion, muscle or joint pain, hyperglycemia, and inflammation. Further, statins are reported to interact with other medicines, increasing the risk of harmful effects, for example, muscle damage. Surgery (*e.g.,* vascular bypass surgery or angioplasty with or without stenting) provides an alternative treatment for atherosclerosis in the case when the atherosclerosis becomes server and causes irreversible ischemia. However, it is known that surgery may result in bleeding, wound haematoma, infection, or even worse, nerve damage.

In the publication of Chen Huei et al, entitled "Gold nanoparticles improve metabolic profile of mice fed a high-fat diet", J. of nanobiotechnology (2018) 16(1), page 11, it is disclosed that gold nanoparticles (AuNPs) are suitable to treat hypercholesrolemia; however, the publication from Chen Huei et al fails to disclose to treat hypercholesrolemia with FANC.

In view of the foregoing, there exists in the related art a need for a novel method for treating hypercholesrolemia in a more safe and efficient manner.

### SUMMARY

This invention is set out in the appended set of claims. References to methods of treatment by therapy in this description are to be interpreted as references to compounds, pharmaceutical compositions and medicaments of the present invention for use in those methods.

As embodied and broadly described herein, one aspect of the disclosure is directed to a method of treating hypercholesterolemia in a subject. The method comprises administering to the subject an effective amount of a dihydrolipoic acid (DHLA)-coated gold nanocluster, which consists of, a gold nanocluster formed by a plurality of gold nanoparticles, and a plurality of DHLAs coated on the gold nanocluster. The DHLA-coated gold nanocluster according to the claimed invention has a particle size ranging from 1 to 10 nm; preferably, ranging from 1 to 5 nm. In some specific examples of the present disclosure, the particle size of the DHLA-coated gold nanocluster is about 2 nm.

According to some embodiments of the present disclosure, the effective amount is about 0.001-10 mg/kg body weight per day; preferably, about 0.01-1 mg/kg body weight per day; more preferably, about 0.01-0.1 mg/kg body weight per day. According to the claimed invention, the DHLA-coated gold nanocluster is administered to the subject in the amount of 0.01-0.1 mg/kg body weight per day. According to one specific example of the present disclosure, the DHLA-coated gold nanocluster is administered to the subject daily for 56 consecutive days.

The subject is a mammal. Preferably, the subject is a human.

Many of the attendant features and advantages of the present disclosure will becomes better understood with reference to the following detailed description considered in connection with the accompanying drawings.

### BRIEF DESCRIPTION OF THE DRAWINGS

The present description will be better understood from the following detailed description read in light of the accompanying drawings, where:
Fig. 1 is a schematic presentation of the present DHLA-coated gold nanocluster prepared in accordance with one embodiment of the present disclosure.
Fig. 2 is a histogram depicting the percentage of the area of atherosclerotic lesion according to Example 1 of the present disclosure. n=6 in each group. *, p<0.05; **, P<0.001.
Fig. 3 is a histogram depicting the serum level of total cholesterol in mice administered with specified treatments according to Example 2 of the present disclosure. n=6 in each group. *, p<0.05; **, P<0.001.
Figs. 4A and 4B are histograms respectively depicting the expression level of malondialdehyde (MDA) and 4-hydroxynonenal (4-HNE) in mice administered with specified treatments according to Example 2 of the present disclosure. n=6 in each group. *, p<0.05; **, P<0.001.
Figs. 5A to 5C are histograms respectively depicting the number of macrophages attached human aortic endothelial cells (HAECs; Fig. 5A), and the expression level of adhesion molecules ICAM-1 (Fig. 5B) and VCAM-1 (Fig. 5C) on HAECs according to Example 3 of the present disclosure. n=3 in each group. *, p<0.05; **, P<0.001.

### DETAILED DESCRIPTION OF THE INVENTION

The detailed description provided below in connection with the appended drawings is intended as a description of the present examples. The description sets forth the functions of the example and the sequence of steps for constructing and operating the example.

### I. Definition

For convenience, certain terms employed in the specification, examples and appended claims are collected here. Unless otherwise defined herein, scientific and technical terminologies employed in the present disclosure shall have the meanings that are commonly understood and used by one of ordinary skill in the art. Also, unless otherwise required by context, it will be understood that singular terms shall include plural forms of the same and plural terms shall include the singular. Specifically, as used herein and in the claims, the singular forms "a" and "an" include the plural reference unless the context clearly indicates otherwise. Also, as used herein and in the claims, the terms "at least one" and "one or more" have the same meaning and include one, two, three, or more.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in the respective testing measurements. Also, as used herein, the term "about" generally means within 10%, 5%, 1%, or 0.5% of a given value or range. Other than in the operating/working examples, or unless otherwise expressly specified, all of the numerical ranges, amounts, values and percentages such as those for quantities of materials, durations of times, temperatures, operating conditions, ratios of amounts, and the likes thereof disclosed herein should be understood as modified in all instances by the term "about". Accordingly, unless indicated to the contrary, the numerical parameters set forth in the present disclosure and attached claims are approximations that can vary as desired. At the very least, each numerical parameter should at least be construed in light of the number of reported significant digits and by applying ordinary rounding techniques.

The term "administering" or "administration" are used interchangeably herein to refer a mode of delivery, including, without limitation, intraveneously, intramuscularly, intraperitoneally, intraarterially, intracranially, or subcutaneously administering an agent (*e.g.,* a DHLA-coated gold nanocluster) of the present invention.

As used herein, the term "treat," "treating" and "treatment" are interchangeable, and encompasses partially or completely preventing, ameliorating, mitigating and/or managing a symptom, a secondary disorder or a condition associated with or caused by hypercholesterolemia, in which decreasing the level of hypercholesterolemia provides a benefit to the subject having or suspected of having such symptom, disorder or condition. The term "treating" as used herein refers to application or administration of the DHLA-coated gold nanocluster of the present disclosure to a subject, who has a symptom, a secondary disorder or a condition associated with or caused by hypercholesterolemia, with the purpose to partially or completely alleviate, ameliorate, relieve, delay onset of, inhibit progression of, reduce severity of, and/or reduce incidence of one or more symptoms, secondary disorders or features associated with or caused by hypercholesterolemia. Symptoms, secondary disorders, and/or conditions associated with or caused by hypercholesterolemia include, but are not limited to, coronary heart disease, angina, heart attack, stroke, TIA, and PADs. Treatment may be administered to a subject who exhibits only early signs of such symptoms, disorder, and/or condition for the purpose of decreasing the risk of developing the symptoms, secondary disorders, and/or conditions associated with or caused by hypercholesterolemia. Treatment is generally "effective" if one or more symptoms or clinical markers are reduced as that term is defined herein. Alternatively, a treatment is "effective" if the progression of a symptom, disorder or condition is reduced or halted.

The term "effective amount" as referred to herein designate the quantity of a component which is sufficient to yield a desired response. For therapeutic purposes, the effective amount is also one in which any toxic or detrimental effects of the component are outweighted by the therapeutically beneficial effects. An effective amount of an agent is not required to cure a disease or condition but will provide a treatment for a disease or condition such that the onset of the disease or condition is delayed, hindered or prevented, or the disease or condition symptoms are ameliorated. The effective amount may be divided into one, two, or more doses in a suitable form to be administered at one, two or more times throughout a designated time period. The specific effective or sufficient amount will vary with such factors as the particular condition being treated, the physical condition of the patient (*e.g.,* the patient's body mass, age, or gender), the type of mammal or animal being treated, the duration of the treatment, the nature of concurrent therapy (if any), and the specific formulations employed and the structure of the compounds or its derivatives. Effective amount may be expressed, for example, in grams, milligrams or micrograms or as milligrams per kilogram of body weight (mg/Kg). Alternatively, the effective amount can be expressed in the concentration of the active component (*e.g.,* the DHLA-coated gold nanocluster of the present disclosure), such as molar concentration, mass concentration, volume concentration, molality, mole fraction, mass fraction and mixing ratio. Persons having ordinary skills could calculate the human equivalent dose (HED) for the medicament (such as the present DHLA-coated gold nanocluster) based on the doses determined from animal models. For example, one may follow the guidance for industry published by US Food and Drug Administration (FDA) entitled "Estimating the Maximum Safe Starting Dose in Initial Clinical Trials for Therapeutics in Adult Healthy Volunteers" in estimating a maximum safe dosage for use in human subjects.

The term "hypercholesterolemia" as used herein refers to any medical condition wherein blood cholesterol levels are elevated above the clinically recommended levels. For example, when the blood cholesterol levels are determined by measuring the levels of low density lipoproteins (LDLs), then hypercholesterolemia may exist if the measured LDL levels are above, for example, approximately 75 mg/dl. Alternatively, when the blood cholesterol levels are determined by measuring the levels of free plasma cholesterol, then hypercholesterolemia may exist if the measured free cholesterol levels are above, for example, approximately 200-220 mg/dl.

As used herein, a "pharmaceutically acceptable" component is one that is suitable for use with humans and/or animals without adverse side effects (such as toxicity, irritation and/or allergic response) commensurate with a reasonable benefit/risk ratio.

The term "excipient" as used herein means any inert substance (such as a powder or liquid) that forms a vehicle or carrier for the DHLA-coated gold nanocluster of the present disclosure. The excipient may be any commercially available excipient as long as it is generally safe and non-toxic for the subject.

The term "subject" refers to a mammal including the human species that is treatable with the DHLA-coated gold nanocluster, medicament, pharmaceutical composition, or method of the present invention. The term "subject" is intended to refer to both the male and female gender unless one gender is specifically indicated.

### II. Description of The Invention

The present disclosure is based, at least in part, on the discovery that DHLA-coated gold nanoclusters are useful in treating atherosclerosis via reducing the level of cholesterol, oxidative stress, and vascular inflammation. Accordingly, the practices of the present disclosure hereinafter described in detail with respect to the use of the DHLA-coated gold nanoclusters in the treatment of hypercholesterolemia.

The DHLA-coated gold nanoclusters used in the present disclosure are known to the skilled practitioner as well as the process for their production (Lin et al., ACS Nano (2009); 3: 395-401); hence no further explanations are necessary with respect to their preparation. The DHLA-coated gold nanoclusters have a fluorescent emission at 650 nm under an excitation wavelength at approximately 420 nm, hence will emit wavelength ranged from red to near infrared. Each gold nanocluster has a particle size ranging from 1 to 10 nm, more preferably from 1 to 5 nm. In certain working examples, the particle size of the gold nanocluster is about 2 nm. The dimension discussed above related to the gold nanoparticle of the present disclosure is in dried state, however, it is of advantage if the gold nanocluster used in the present disclosure is watersoluble or at least dispersible in aqueous medium and/or water; the hydrodynamic size of the dried nanocluster can be significantly larger than the dried size due to the coupling of surrounding solvent molecule such as water. In one specific embodiment example, the gold nanocluster has a hydrodynamic size corresponds to 1 to 30 kDa polyethylene glycol (PEG).

In one aspect of the present disclosure, the DHLA-coated gold nanoclusters are used to prepare a pharmaceutical composition or a medicament for treating hypercholesterolemia. The pharmaceutical composition or medicament comprises the DHLA-coated gold nanoclusters, and optionally, a pharmaceutically acceptable excipient.

The DHLA-coated gold nanoclusters may be present in the medicament or pharmaceutical composition at a level of about 0.01% to 99.9% by weight, based on the total weight of the medicament or pharmaceutical composition. In some embodiments, the DHLA-coated gold nanoclusters are present in the medicament or pharmaceutical composition at a level of at least 0.1% by weight, based on the total weight of the medicament or pharmaceutical composition. In certain embodiments, the DHLA-coated gold nanoclusters are present in the medicament or pharmaceutical composition at a level of at least 5% by weight, based on the total weight of the medicament or pharmaceutical composition. In still other embodiments, the DHLA-coated gold nanoclusters are present in the medicament or pharmaceutical composition at a level of at least 10% by weight, based on the total weight of the medicament or pharmaceutical composition. In still yet other embodiments, the DHLA-coated gold nanoclusters are present in the medicament or pharmaceutical composition at a level of at least 25% by weight, based on the total weight of the medicament or pharmaceutical composition.

For the application of the present invention, the DHLA-coated gold nanoclusters of the present disclosure may be manufactured into desired formulations, such as tablets, sugar-coated tablets, pills, granules, aerosols, syrups, emulsions, suspensions, solutions, ointments, creams or gels or any kind, in particular by using inert, essentially nontoxic, pharmaceutically suitable excipients or carriers.

Also disclosed herein is a method of treating hypercholesterolemia in a subject. The method comprises administering to the subject an effective amount of the present DHLA-coated gold nanoclusters, or the medicament or pharmaceutical composition comprising the same.

The present DHLA-coated gold nanoclusters, medicament or pharmaceutical composition may be administered systematically or locally. Any customary forms of administration are suitable for administering the DHLA-coated gold nanoclusters of the present disclosure. Administration may be carried out, for example, orally, lingually, sublingually, buccally, rectally or parenterally (*i.e.,* intravenously, intraarterially, intracardially, intracutaneously, subcutaneously, transdermally, intraperitoneally or intramuscularly).

According to some examples of the present disclosure, the subject is a mouse. To elicit a therapeutic effect on mice, the present DHLA-coated gold nanoclusters is administered to the subject in the amount of about 0.01 to 150 mg/Kg body weight per day; preferably, about 0.1 to 15 mg/Kg body weight per day; more preferably, about 0.1-1.5 mg/Kg body weight per day. According to one working example, the present DHLA-coated gold nanoclusters are administered to the subject in the amount of 0.57 mg/Kg body weight per day.

A skilled artisan may readily determine the human equivalent dose (HED) of the present DHLA-coated gold nanoclusters, based on the doses determined from animal studies provided in working examples of this application. Accordingly, the amount of the present DHLA-coated gold nanoclusters suitable for use in a human subject may be in the range of 0.001-10 mg/Kg body weight per day; for example, 0.001. 0.002, 0.003, 0.004, 0.005, 0.006, 0.007, 0.008, 0.009, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5, or 10 mg/kg body weight per day. Preferably, the amount of the present DHLA-coated gold nanoclusters for treating a human subject is about 0.01-1 mg/Kg body weight per day. More preferably, the amount of the present DHLA-coated gold nanoclusters for treating a human subject is about 0.01-0.1 mg/Kg body weight per day.

Alternatively, the effective amount of the present DHLA-coated gold nanoclusters may vary with clinical factors, such as the particular condition being treated, the severity of the condition, the individual patient parameters (including age, physical condition, size, gender and weight), the duration of the treatment, the nature of concurrent therapy (if any), the specific route of administration and like factors within the knowledge and expertise of the health practitioner.

The skilled artisan or health practitioner may adjust the dosing regimen of the DHLA-coated gold nanoclusters in accordance with various factors, such as age, gender, weight, and other treatments (if any). For example, the present DHLA-coated gold nanoclusters may be administered to the subject 1-7 times per week (*e.g.,* 1, 2, 3, 4, 5, 6 or 7 times per week) for 1, 2, 3, 4 or more consecutive weeks. Alternatively, the present DHLA-coated gold nanoclusters may be administered to the subject 1-10 times (*e.g*., 1, 2, 3, 4, 5, 6, 7, 8, 9 or 10 times) for every 2 weeks, every 4 weeks, every 5 weeks, every 6 weeks, every 7 weeks, every 8 weeks, every 9 weeks, or every 10 weeks; or once every month, every 2 months, or every 3 months, or longer. Preferably, the present DHLA-coated gold nanoclusters are administered to the subject daily for at least 14 days (*i.e.,* 2 weeks), for example, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or more days. More preferably, the present DHLA-coated gold nanoclusters are administered to the subject daily for at least 28 days (*i.e.,* 4 weeks). According to one working example of the present disclosure, the present DHLA-coated gold nanoclusters are administered to the subject daily for 56 days (*i.e.,* 8 weeks) so as to produce the therapeutic effect.

Basically, the subject is a mammal, for example, a human, a mouse, a rat, a hamster, a guinea pig, a rabbit, a dog, a cat, a cow, a goat, a sheep, a monkey, and a horse. Preferably, the subject is a human.

As would be appreciated, the present method can be applied to the subject, alone or in combination with additional therapies that have some beneficial effects on the treatment of hypercholesterolemia or atherosclerosis, for example, statins. Depending on the intended purpose, the present method can be applied to the subject before, during, or after the administration of the additional therapies.

The following Examples are provided to elucidate certain aspects of the present invention and to aid those of skilled in the art in practicing this invention. These Examples are in no way to be considered to limit the scope of the invention in any manner. Without further elaboration, it is believed that one skilled in the art can, based on the description herein, utilize the present invention to its fullest extent.

### EXAMPLE

### Materials and Methods

### Preparation of DHLA-Coated Gold Nanoclusters (FANC)

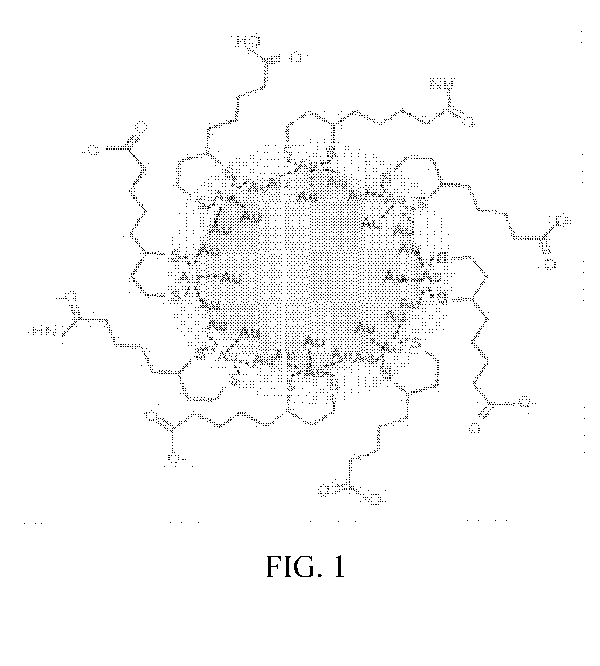

Fluorescent gold nanoclusters used in this study were prepared as previously described (Lin et al., ACS Nano (2009); 3: 395-401). Briefly, 6-nm gold nanoparticles stabilized with didodecyldimethylammonium bromide (AuNP@DDAB) were synthesized via an established single-phase reaction (Jana and Peng, J Am Chem Soc (2003); 125: 14280-14281). The composition of AuNP@DDAB is schematically depicted in Fig. 1. Subsequent further dropwise addition of gold precursor solution (AuCl₃ in DDAB-toluene solution) caused a gradual loss of plasmon absorption until the solution turned yellowish transparent. Ligand exchange was performed by adding the as-prepared nanoclusters to the reduced lipoic acid (DHLA, dihydrolipoic acid), which was freshly reduced by tetrabutylammonium borohydride (TBAB) with a molar ratio of lipoic acid to TBAB = 4:1. This leaded to dark-brown nanocluster agglomerates in the resulting mixture, and additional UV lamp exposure (365nm, 30 mins) was treated to condense the agglomerates. After discarding the supernatant, nanoclusters were re-dispersed in methanol and precipitated again in additional chloroform so as to remove free surfactants. The dried nanoclusters precipitate were dispersed in borate buffer (pH 9). Further purification was achieved by three runs of ultracentrifugation (110,000 rpm) to remove excess DHLA. Gold nanoclusters was collected, and phosphate-buffered saline (PBS) was changed through a centrifuge filter of 30 kDa molecular weight cut-off (MWCO), leading to a colloidally stable transparent solution of NCs without plasmon peak. The concentration of gold nanoclusters was measured by the extinction coefficient of about 450,000 M⁻¹cm⁻¹ at 420 nm.

### Adhesion assay

Human aortic endothelial cells (HAECs) were maintained in endothelial growth medium. Cells were seeded onto 1% gelatin-coated plastic ware or 2% gelatin-coated glass coverslips in a density of 10,000 cells/cm² and maintained at 37°C in a humidified incubator with 95% air and 5% CO₂ atmosphere.

The HAECs were mixed with the specified concentration (*i.e*., 0 nM, 50 nM, or 100 nM) of the DHLA-coated gold nanoclusters for 3 days followed by stimulating with 100 ng/ml lipopolysaccharides (LPS) for 24 hours. Activated HAECs were incubated with Calcein AM-loaded monocytes for adhesion assay. After two-hour incubation, unbound monocytes were washed out and cell images were recorded by florescence microscope.

### Animal Experiment

Four-week-old *ApoE*^{*-*/*-*}mice and wild type *C57BL*/*6* mice fed either normal chow or a pelleted Western diet (W. diet) (containing 0.21% cholesterol) were treated with FANC (0.57 mg/kg daily for 56 days) or placebo (PBS). The mice were divided into three groups as follows: Group 1, wild type mice fed with regular chow; Group 2, *ApoE*^{*-*/*-*} mice fed regular chow and treated with PBS; Group 3, *ApoE*^{*-*/*-*} mice fed Western diet and treated with FANC.

### Detection and Quantification of Atherosclerotic Lesions

The aortas were respectively isolated from ApoE-deficient and wild type mice administered with specified treatments as described in Animal Experiment followed by the analysis of Sudan IV staining, a red beta-naphthol diazo dye for staining lipid-containing substances (*e.g.*, triglycerides, lipids and lipoproteins) in cells and tissues. The histology and pathology of the staining aortas were then detected by microscopy, and the areas of the atherosclerotic lesions were determined by software.

### Analysis of cholesterol, MDA and 4-HNE

For the purpose of evaluating whether the present DHLA-coated gold nanoclusters affects the level of cholesterol and oxidative stress, the sera were respectively isolated from *ApoE-*deficient mice 56 days post-treatment. The serum level of total cholesterol was examined by cholesterol test kit, and the expression levels of MDA and 4-HNE, two indicators of oxidative stress, were measured by enzyme-linked immunosorbent assay (ELISA).

The following examples 1 to 3 are not according to the claimed invention and are present for illustration purposes only.

### Reference example 1 Anti-Atherosclerotic Effect of DHLA-Coated Gold Nanoclusters

The effect of the DHLA-coated gold nanoclusters on atherosclerosis was evaluated in this example, in which the *ApoE*-deficient mice fed normal chow (serving as a control group) or Western diet (*i.e.,* a high-fat diet) were administered with PBS or the DHLA-coated gold nanoclusters.

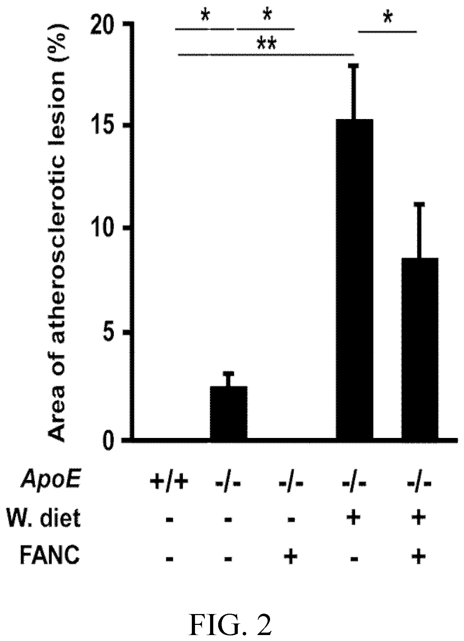

Compared to the *ApoE*-deficient mice fed normal chow, the atheromatous plaques of which covered 2.57± 0.5 % of the luminal area, a high-fat diet significantly increased the burden of atherosclerotic plaques in *ApoE*-deficient mice, in which the atheromatous plaques increased more than 6 folds and covered 15.35 ± 2.6 % of the luminal area (Fig. 2). It is noted that the administration of the DHLA-coated gold nanoclusters (designated as "FANC" in Fig. 2) markedly reduced the burden of atherosclerotic plaques (Fig. 2). In the *ApoE*-deficient mice fed normal chow and treated with the DHLA-coated gold nanoclusters, the atheromatous plaques were virtually invisible. The *ApoE*-deficient mice fed a high-fat diet exhibited a similar result, in which the administration of the DHLA-coated gold nanoclusters decreased the burden of atheromatous plaques by about 43.3 % in comparison with the PBS treatment group, and the area of the atheromatous plaques reduced to 8.70 ± 2.5 % of the luminal area (Fig. 2).

The data demonstrated that the present DHLA-coated gold nanoclusters exhibits a therapeutic effect on atherosclerosis that provides a therapeutic benefit to various atherosclerotic cardiovascular diseases.

### Example 2 Inhibitory Effect of DHLA-Coated Gold Nanoclusters on Serum Cholesterol And Oxidative Stress (in as far as relating to the inhibition of oxidative stress, this example represents a reference example)

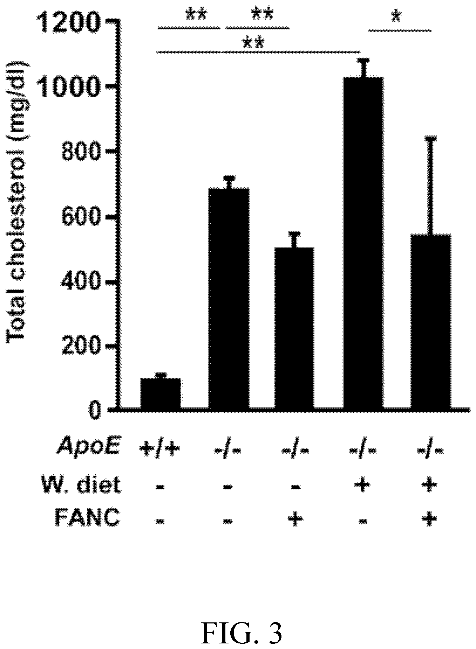

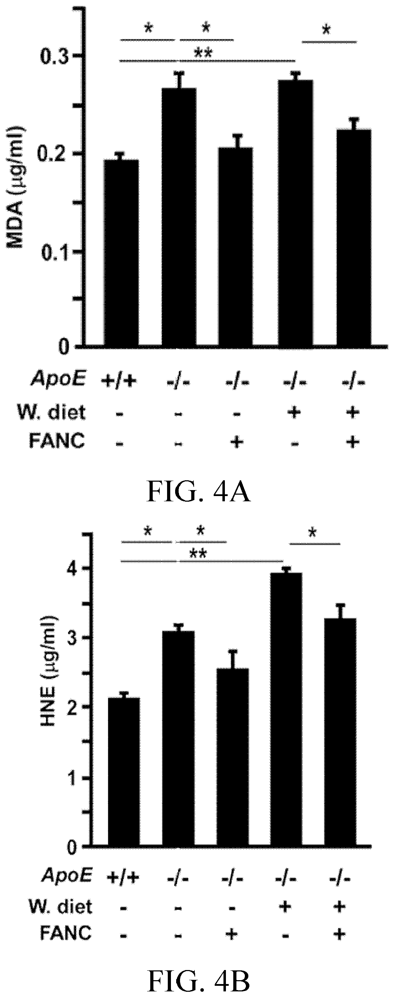

Whether the present DHLA-coated gold nanoclusters affect the expression or level of cholesterol and oxidative stress was examined in this example. The results were respectively depicted in Figs. 3 and 4.

The data of Fig. 3 indicated that compared to the normal chow, a high-fat diet obviously increased the serum level of total cholesterol in ApoE-deficient mice (normal chow: 567.7 ± 32.5 mg/dL; high-fat diet: 1020.0 ± 55.0 mg/dL). The administration of the DHLA-coated gold nanoclusters (designated as "FANC" in Fig. 3) markedly reduced the cholesterol level, in which in the *ApoE*-deficient mice fed normal chow and treated with the DHLA-coated gold nanoclusters, the serum level of total cholesterol decreased to 501.0 ± 46. 7 mg/dL; while in the *ApoE*-deficient mice fed high-fat diet and treated with the DHLA-coated gold nanoclusters, the serum level of total cholesterol had a 48.1% of decrement, being reduced to 529.0 ± 300.0 mg/dL (Fig. 3).

In addition to cholesterol, the DHLA-coated gold nanoclusters also exhibited an inhibitory effect on the expression of MDA and 4-HNE. The data of Figs. 4A and 4B respectively indicated that compared to wild-type mice (*ApoE*^{*+*/*+*} mice) fed normal chow, both serum levels of MDA and 4-HNE increased in the *ApoE*-deficient mice fed either normal chow or high-fat diets. The administration of the DHLA-coated gold nanoclusters (designated as "FANC" in Figs. 4A and 4B) significantly reduced the serum levels of these oxidative stress indicators as compared to the mice fed the same diet with PBS treatment (Figs. 4A and 4B).

These results demonstrated that the DHLA-coated gold nanoclusters of the present disclosure effectively inhibits the level of cholesterol and oxidative stress in a subject.

### Reference example 3 Effect of DHLA-Coated Gold Nanoclusters on Adhesion Molecules

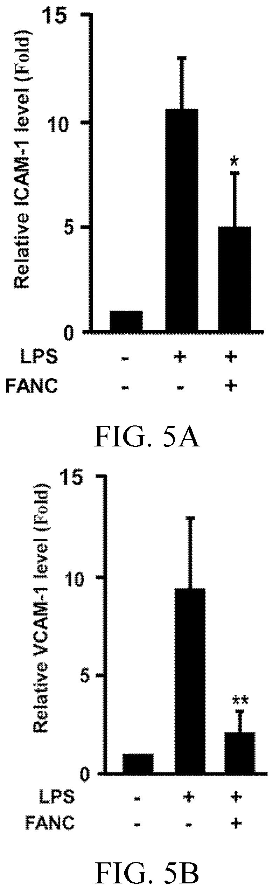

Since the adhesion of inflammatory cells (*e.g*., macrophages) to vascular wall plays a role in mediating vascular inflammation, which further leads to the formation and progression of atherosclerosis, the HAECs stimulated with LPS were employed in this example so as to evaluate the effect of the present DHLA-coated gold nanoclusters (designated as "FANC" in Figs. 5A to 5C) on the vascular inflammation. The results were respectively depicted in Figs. 5A to 5C.

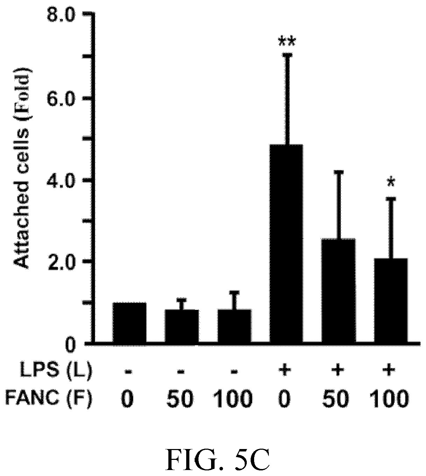

As depicted in Figs. 5A and 5B, LPS induced the expression of adhesion molecules (*i.e.,* ICAM-1 and VCAM-1, two adhesion molecules mediating the vascular inflammation) on HAECs, while the administration of the DHLA-coated gold nanoclusters significantly reduced the LPS-induced expression. Further, the present DHLA-coated gold nanoclusters also reduced the number of macrophages adhesive to HAECs in a dose-dependent manner (Fig. 5C).

In conclusion, the present disclosure demonstrates that the DHLA-coated gold nanoclusters is effective on the treatment of atherosclerosis via reducing the level of cholesterol, oxidative stress, and adhesion molecules (*e.g*., ICAM-1 and VCAM-1). Thus, the DHLA-coated gold nanoclusters may be used to prepare medicaments for treating various ASCVDs.

## Claims

1. A dihydrolipoic acid (DHLA) - coated gold nanocluster for use in the treatment of hypercholesterolemia in a subject, wherein the DHLA-coated gold nanocluster has a particle size ranging from 1 to 10 nm and consists of a gold nanocluster formed by a plurality of gold nanoparticles and a plurality of DHLAs coated on the gold nanocluster wherein the DHLA-coated gold nanocluster is administered to the subject in the amount of 0.01-0.1 mg/kg body weight per day.

2. The DHLA - coated gold nanocluster for use of claim 1, wherein the DHLA-coated gold nanocluster has a particle size ranging from 1 to 5 nm.

3. The DHLA - coated gold nanocluster for use of claim 2, wherein the DHLA-coated gold nanocluster has a particle size of 2 nm.

4. The DHLA - coated gold nanocluster for use of claim 1, wherein the DHLA - coated gold nanocluster is administered to the subject daily for at least 56 consecutive days.

5. The DHLA - coated gold nanocluster for use of claim 1, wherein the subject is a human.

## Patentansprüche

1. Dihydroliponsäure (DHLA)-beschichteter Gold-Nanocluster zur Verwendung in der Behandlung von Hypercholesterinämie in einem Subjekt, wobei der DHLAbeschichtete Gold-Nanocluster eine Partikelgröße in einem Bereich von 1 bis 10 nm hat und aus einem Gold-Nanocluster, der durch eine Vielzahl von Gold-Nanopartikeln gebildet wird, und einer Vielzahl von DHLAs besteht, die auf den Gold-Nanocluster aufgebracht sind, wobei der DHLA - beschichtete Gold-Nanocluster dem Subjekt in einer Menge von 0,01-0,1 mg/kg Körpergewicht pro Tag verabreicht wird.

2. DHLA - beschichteter Gold-Nanocluster zur Verwendung nach Anspruch 1, wobei der DHLA- beschichtete Gold-Nanocluster eine Partikelgröße in einem Bereich von 1 bis 5 nm hat.

3. DHLA - beschichteter Gold-Nanocluster zur Verwendung nach Anspruch 2, wobei der DHLA- beschichtete Gold-Nanocluster eine Partikelgröße von 2 nm hat.

4. DHLA - beschichteter Gold-Nanocluster zur Verwendung nach Anspruch 1, wobei der DHLA- beschichtete Gold-Nanocluster dem Subjekt täglich für mindestens 56 aufeinanderfolgende Tage verabreicht wird.

5. DHLA - beschichteter Gold-Nanocluster zur Verwendung nach Anspruch 1, wobei das Subjekt ein Mensch ist.

## Revendications

1. Nano-agrégat d'or enrobé d'acide dihydrolipoïque (DHLA) destiné au traitement de l'hypercholestérolémie chez un sujet, dans lequel le nano-agrégat d'or enrobé de DHLA présente une taille de particules allant de 1 à 10 nm et consiste en un nano-agrégat d'or formé par une pluralité de nanoparticules d'or et une pluralité de DHLA enrobés sur le nano-agrégat d'or, dans lequel le nano-agrégat d'or enrobé de DHLA est administré au patient à une dose de 0,01 à 0,1 mg/kg de poids corporel par jour.

2. Nano-agrégat enrobé de DHLA pour une utilisation selon la revendication 1, dans lequel le nano-agrégat d'or enrobé de DHLA présente une taille de particules allant de 1 à 5 nm.

3. Nano-agrégat d'or enrobé de DHLA pour une utilisation selon la revendication 2, dans lequel le nano-agrégat d'or enrobé de DHLA présente une taille de particules de 2 nm.

4. Nano-agrégat d'or enrobé de DHLA pour une utilisation selon la revendication 1, dans lequel le nano-agrégat d'or enrobé de DHLA est administré au sujet quotidiennement pendant au moins 56 jours consécutifs.

5. Nano-agrégat d'or enrobé de DHLA pour une utilisation selon la revendication 1, dans lequel le sujet est un humain.
